# EUROPEAN PATENT SPECIFICATION

(11) **EP 3 020 343 B1**
(45) Date of publication and mention of the grant of the patent: **19.06.2019**
(21) Application number: 13889028.0
(22) Date of filing: 08.07.2013
(51) Int. Cl.: A61B 17/11

(54) **APPLICATION TOOL**
ANWENDUNGSWERKZEUG
OUTIL D'APPLICATION

(43) Date of publication of application: 18.05.2016
(73) Proprietor: Terumo Kabushiki Kaisha, Tokyo 151-0072 (JP)
(72) Inventor: SOMA, Katsuaki, Fujinomiya-shi Shizuoka 418-0015 (JP)
(74) Representative: Casalonga
(86) International application number: PCT/JP2013/068622
(87) International publication number: WO 2015/004709

(56) References cited:
- JP-A- 2009 131 590
- JP-A- 2009 131 590
- JP-A- 2009 213 786
- JP-A- 2010 051 696
- JP-A- 2010 051 696
- JP-A- 2011 056 019
- JP-A- 2012 110 514
- US-A1- 2006 280 690

## Description

### Technical Field

The present invention relates to an applicator.

### Background Art

Heretofore, a method has been known in which two or more kinds of liquids are mixed and jetted to an affected area or the like to form an anti-adhesion material or a biological tissue adhesive, and an applicator for this purpose has been developed.

Such an applicator as just mentioned has a configuration wherein components which solidify when mixed together, for example, a solution containing thrombin and a solution containing fibrinogen, are fed to a position in the vicinity of an affected area in a separated state from each other, to be applied while being mixed in the affected area.

As a conventional applicator, there is one that includes two syringes containing individually different kinds of liquids and a nozzle for mixing liquids supplied from the syringes and jetting the mixed liquid (see, for example, Patent Document 1). The applicator described in Patent Document 1 has a configuration in which the nozzle is connected to a gas supplying source for supplying a sterile gas, and the liquids are jetted together with the sterile gas. More specifically, this nozzle has a double tube structure composed of two inner tubes through which the liquids from each of the syringes flow, and an outer tube in which the two inner tubes are inserted and which allows the gas to flow through a gap between the outer tube and the inner tubes.

In addition, distal portions of the inner tubes constitute a confluence portion where they join, and the liquids are mixed with each other in the confluence portion, to form a mixture. The confluence portion is configured from a gas-permeable membrane which is permeable to gas but impermeable to liquid. Accordingly, when plungers of the syringes are pushed to perform a liquid jetting operation, the mixture can be jetted together with the sterile gas flowing into the confluence portion through the gas-permeable membrane.

In the nozzle configured as just mentioned, upon stopping of the liquid jetting operation, the mixture in the confluence portion may be excessively pushed back, or may be caused to retract (flow back), depending on the magnitude of the pressure of the gas flowing into the confluence portion. In this case, depending on the composition of the mixture, the mixture may solidify, resulting in clogging. If the jetting operation is attempted again in the state where clogging has occurred, the solidified mixture would obstruct the jetting of the liquid from the nozzle. Thus, there have been cases where a jetting operation is difficult to carry out due to such a situation.

### Prior Art Document

### Patent Document

Patent Document 1: Japanese Patent No. 5147465. Patent document JP2010 051696 discloses features falling under the preamble of claim 1. This document is considered the prior art being closest to the subject-matter of the present invention.

### Summary of Invention

### Technical Problem

It is an object of the present invention to provide an applicator which can prevent clogging of a nozzle from occurring when an applying operation is stopped.

### Technical Solution

The above object can be achieved by the present invention. The invention is defined in claim 1. Further embodiments of the invention are defined in the dependent claims.

### Advantageous Effect

According to the present invention, when the applying operation is stopped, the mixed liquid in the confluence portion is discharged assuredly by the gas flowing in through the gas-permeable tube provided upstream of the confluence portion. Therefore, the mixed liquid can be prevented from remaining in the confluence portion, and clogging of the confluence portion (jet port) can be prevented from occurring.

### Brief Description of Drawings

FIG. 1 is a perspective view showing a preferable embodiment of an applicator of the present invention.
FIG. 2 is a longitudinal sectional view of a nozzle possessed by the applicator shown in FIG. 1.
FIG. 3 is an enlarged longitudinal sectional view of a distal portion of the nozzle possessed by the applicator shown in FIG. 1 (view depicting the nozzle during an applying operation).
FIG. 4 is an enlarged longitudinal sectional view of a distal portion of the nozzle possessed by the applicator shown in FIG. 1 (view depicting the nozzle after a stopped condition of the applying operation).
FIG. 5 is an enlarged longitudinal sectional view of a distal portion of the nozzle possessed by the applicator shown in FIG. 1 (view depicting the nozzle after a stopped condition of the applying operation).
FIG. 6 is a sectional view taken along line A-A of FIG. 2.

### Mode for Carrying Out the Invention

An applicator of the present invention will be described in detail below, on the basis of a preferable embodiment illustrated in the accompanying drawings.

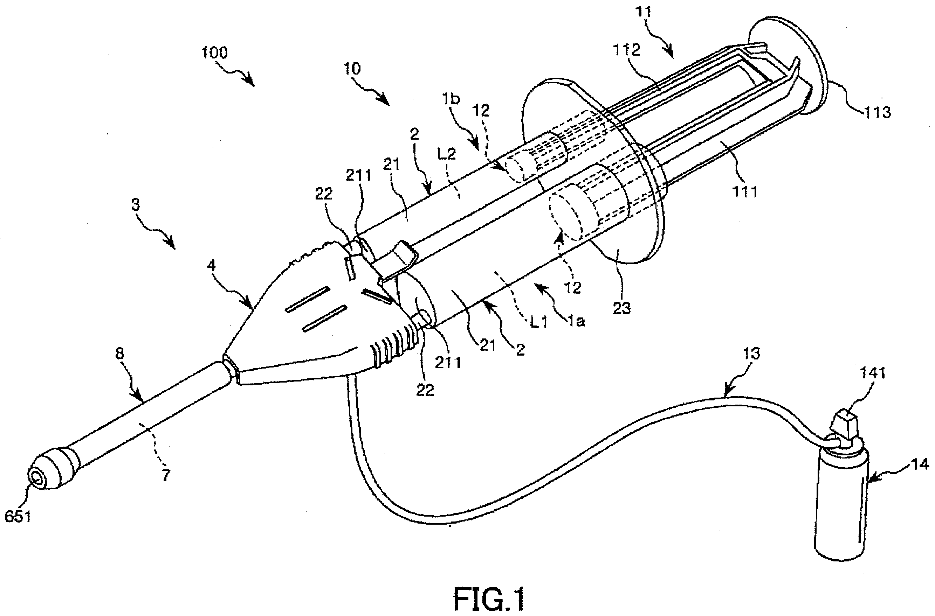

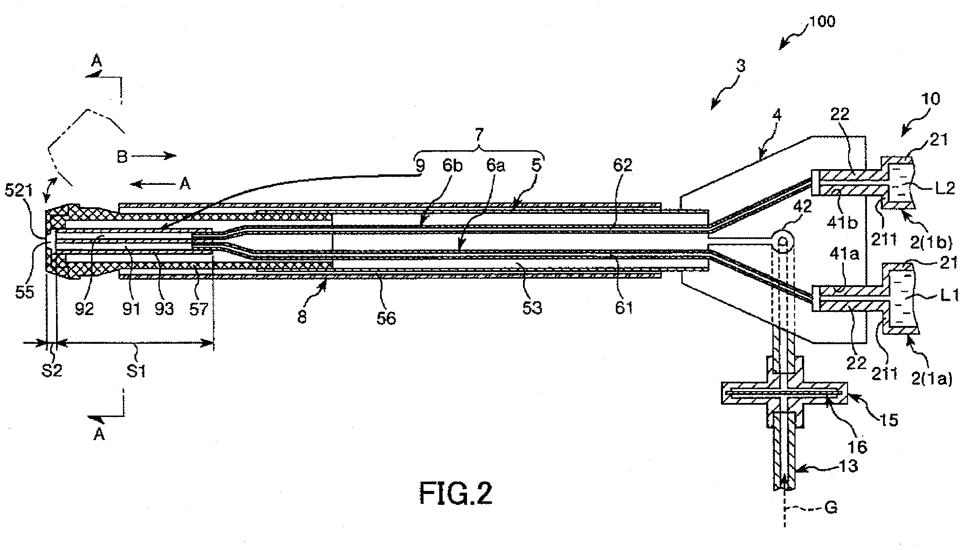

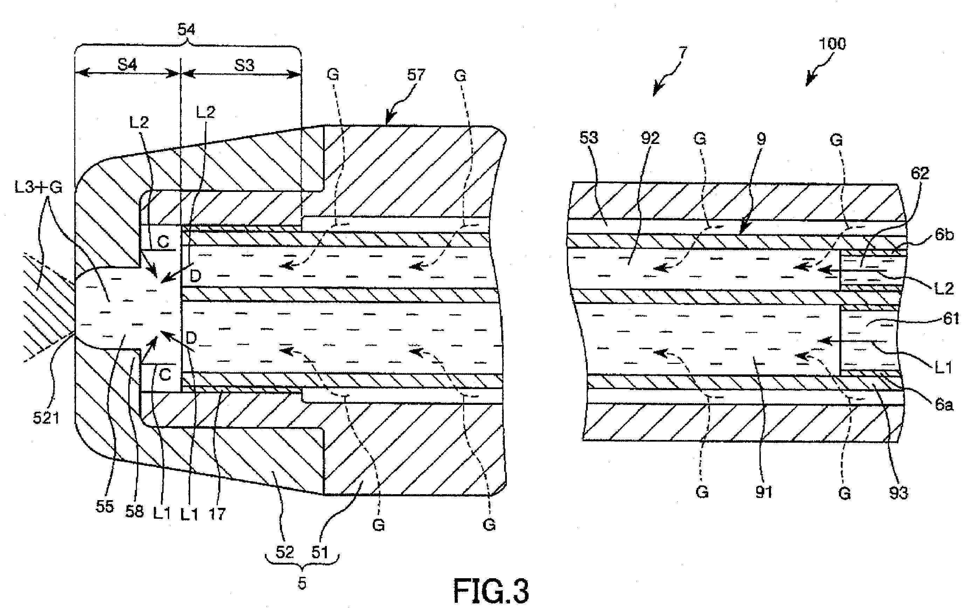

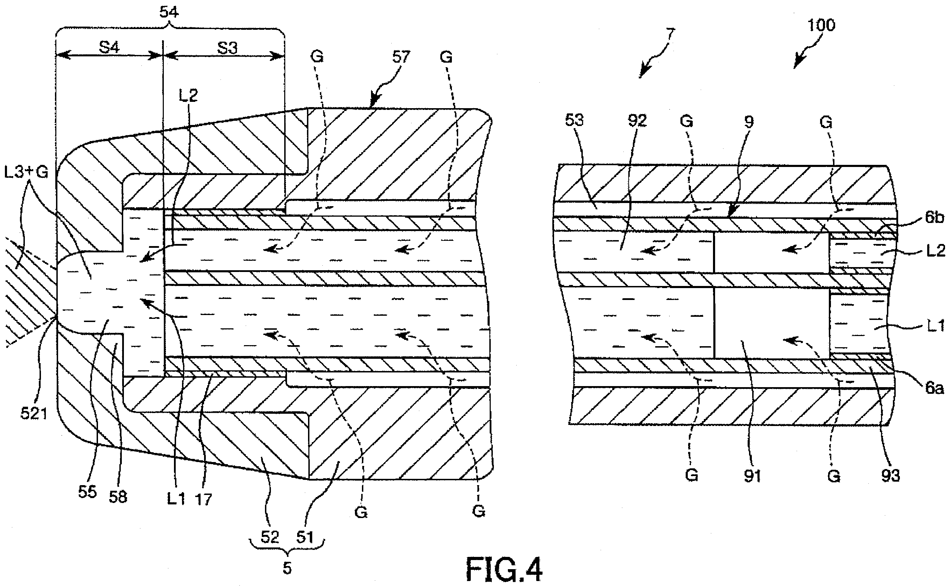

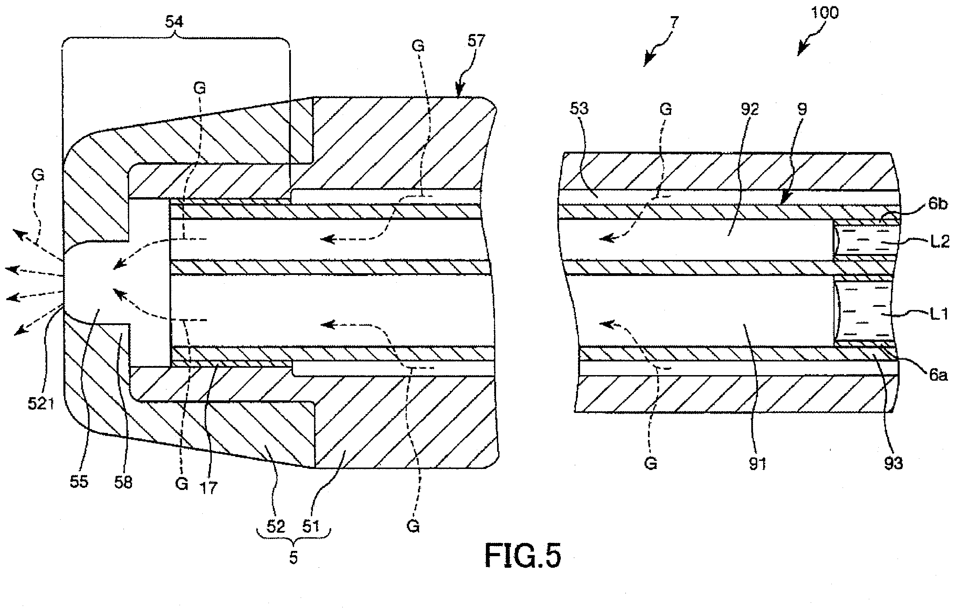

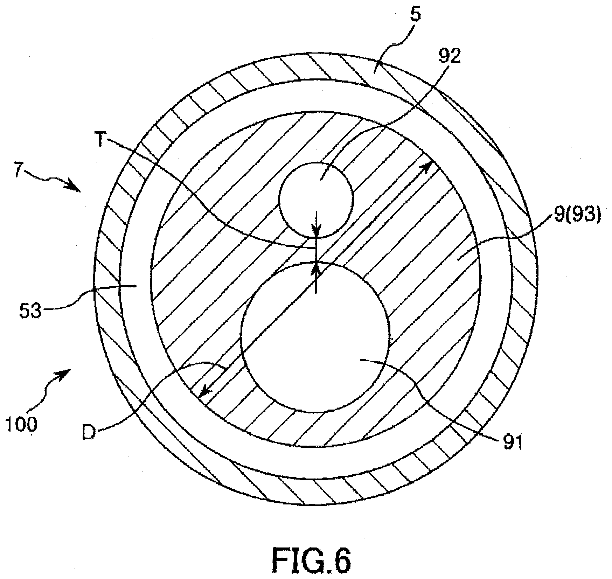

FIG. 1 is a perspective view showing a preferable embodiment of an applicator of the present invention; FIG. 2 is a longitudinal sectional view of a nozzle possessed by the applicator shown in FIG. 1; FIG. 3 is an enlarged longitudinal sectional view of a distal portion of the nozzle possessed by the applicator shown in FIG. 1 (view depicting the nozzle during an applying operation); FIGS. 4 and 5 are enlarged longitudinal sectional views of a distal portion of the nozzle possessed by the applicator shown in FIG. 1 (views depicting the nozzle after a stopped condition of the applying operation); and FIG. 6 is a sectional view taken along line A-A of FIG. 2.

Note that in the following description, for convenience of explanation, the right side in FIGS. 1 to 4 will be referred to as "proximal (rear)" or "upstream side," and the left side as "distal (front)" or "downstream side." In addition, in FIGS. 1 to 4, for easier understanding, the nozzle is schematically shown in the state of being reduced in a length direction and exaggerated in a diametric size direction, so that the ratio between the dimension in the length direction and the dimension in the diametric size direction is different from the actual ratio.

An applicator 100 shown in FIG. 1 includes a syringe assembly 10 and a nozzle 3. As illustrated in FIG. 3, the applicator 100 is for an applying operation to mix two kinds of liquids (a first liquid L1 and a second liquid L2) different in liquid composition with each other, and while mixing them, to apply the resulting mixed liquid L3 together with a gas G. The configuration of each of components will be described below.

As depicted in FIG. 1, the syringe assembly 10 is liquid supplying means for collectively supplying the first liquid L1 and the second liquid L2 to the nozzle 3, and includes a syringe 1a and a syringe 1b arranged in parallel and connected to each other.

Note that the syringe 1a is prefilled with the first liquid L1 and the syringe 1b is prefilled with the second liquid L2.

In the case of mixing the first liquid L1 and the second liquid L2 in a predetermined mixing ratio to prepare the mixed liquid L3 and making the mixed liquid L3 a biological tissue adhesive, one of the first liquid L1 and the second liquid L2 may be thrombin and the other agent may be fibrinogen. On the other hand, in the case of making the mixed liquid L3 an anti-adhesion material, one of the agents may be carboxymethyl dextrin modified with succinimidyl group and the other may be a mixture of sodium hydrogen carbonate and sodium carbonate. Note that in the present embodiment, the first liquid L1 is higher than the second liquid L2 in viscosity.

The first liquid L1 and the second liquid L2 in such a combination as this alter, specifically gel (solidify), when mixed with each other. The gelation ensures, for example, that the material obtained upon mixing of the first liquid L1 and the second liquid L2 (hereinafter referred to also as "mixed liquid L3") can reliably remain at a biological tissue (target region) to which it has been applied. Besides, since the mixture assuredly remains at the target region, it can reliably exhibit the function as a biological tissue adhesive or anti-adhesion material at the target region.

The syringe 1a and the syringe 1b are substantially the same in configuration, except for a difference in size, namely, maximum internal volume, and, accordingly, the syringe 1a will be described below on a representative basis.

The syringe 1a includes a syringe outer cylinder 2 and a gasket 12.

The syringe outer cylinder 2 includes a barrel portion 21 in the form of a bottomed cylinder, and a mouth portion (syringe side port portion) 22 formed to project at a bottom portion which is a distal wall portion 211 of the barrel portion 21.

The barrel portion 21 has an inside diameter and an outside diameter which are both constant along the center axis direction of the barrel portion 21. Note that the inside diameter of the barrel portion 21 of the syringe 1a is greater than the inside diameter of the barrel portion 21 of the syringe 1b. Similarly, the outside diameter of the barrel portion 21 of the syringe 1a is also greater than the outside diameter of the barrel portion 21 of the syringe 1b.

In addition, the barrel portion 21 of the syringe 1a and the barrel portion 21 of the syringe 1b are connected to each other at their intermediate portions in the center axis direction through a flange portion 23 having a plate-like shape. By this connection, the positional relationship between the syringe 1a and the syringe 1b is restricted; in other words, a state in which the syringe 1a and the syringe 1b are connected in parallel to each other is maintained.

The mouth portion 22 is a portion which has a tubular shape smaller in diametric size than the barrel portion 21 and which communicates with the barrel portion 21. Through the mouth portion 22, the first liquid L1 is discharged. Note that the mouth portion 22 is disposed at a position eccentric with respect to the center of the distal wall portion 211 of the barrel portion 21. In the present embodiment, the mouth portion 22 of the syringe 1a and the mouth portion 22 of the syringe 1b are equal in outside diameter.

The material constituting the syringe outer cylinder 2 is not particularly limited. For example, resin materials such as polypropylene, cyclic polyolefin, polyesters, poly(4-methylpentene-1), polycarbonate, etc. may be preferably used as the material because these can easily be molded. Note that the constituent material of the syringe outer cylinder 2 is preferably substantially transparent for assuring visibility of the inside.

The gasket 12 is composed of an elastic body having a cylindrical or disk-like shape. The gasket 12 is accommodated in the barrel portion 21 (syringe outer cylinder 2) and is slidable within the barrel portion 21. Besides, a space surrounded by the gasket 12 and the barrel portion 21 can be filled with the first liquid L1. With the gasket 12 moved toward the distal side, starting from the filled state, the first liquid L1 can be discharged through the mouth portion 22.

The material constituting the gasket 12 is not specifically restricted. Examples of the material usable here include elastic materials such as various rubber materials such as silicone rubbers, etc., various thermoplastic elastomers such as polyurethane-based ones, styrene-ethylene-butylene-styrene (SEBS) type styrene-based ones, etc., and mixtures of them.

In addition, the syringe assembly 10 further includes a plunger 11.

The plunger 11 is a member for operating the gaskets 12 collectively. The plunger 11 includes a plunger portion 111 connected to the gasket 12 of the syringe 1a, a plunger portion 112 connected to the gasket 12 of the syringe 1b, and a flange portion 113 serving as an operation section.

The plunger portion 111 is elongate in shape, and its distal portion is connected to the gasket 12 of the syringe 1a. Similarly, the plunger portion 112 is elongate in shape, and its distal portion is connected to the gasket 12 of the syringe 1b. The method for connection here is not particularly limited, and examples of the connecting method include screwing and fitting. Note that the plunger portion 111 is greater than the plunger portion 112 in diametric size (thickness).

The flange portion 113 is plate-like in shape, and the plunger portions 111 and 112 extend distally from a distal surface of the flange portion 113. At the time of operating the applicator 100, for example, a thumb of one hand can be put on the flange portion 113 of the plunger 11, and an index finger and a middle finger of the hand can be put on the flange portion 23 of the syringe outer cylinder 2.

As shown in FIG. 2, the nozzle 3 includes: a base portion 4; a structure 7 having a double tube structure composed of an outer tube 5 and inner tubes 6a and 6b; and a sheath 8.

The base portion 4 is composed of a member which is flat shaped externally. The material constituting the base portion 4 is not particularly limited; for example, the same materials as those for constituting the syringe outer cylinder 2 can be used.

The base portion 4 is provided at a proximal portion thereof with connection portions 41a and 41b. The connection portion 41a is composed of a cylindrical recessed portion, to the inside of which the mouth portion 22 of the syringe 1a is connected liquid-tight. Similarly, the connection portion 41b is composed of a cylindrical recessed portion, to the inside of which the mouth portion 22 of the syringe 1b is connected liquid-tight.

In addition, the base portion 4 is provided with a connection portion 42 at its surface on one side, specifically, its surface serving as a lower surface in a use state of the applicator 100. The connection portion 42 is composed of a cylindrical recessed portion, to which one end portion of a flexible tube 13 is connected liquid-tight. To the other end of the tube 13 is connected a bomb 14.

The bomb 14 has an internal space filled with a high-pressure (compressed) gas G, and can supply the gas G to the applicator 100 (nozzle 3). The bomb 14 is equipped with an on-off valve (cock) 141 capable of controlling the supply and the stop of the supply of the gas G to the applicator 100. When using the applicator 100, the valve 141 is put into an open state. Note that the gas G is not particularly limited, and may be, for example, carbon dioxide. In addition, although the gas G is preferably in a sterile state, the gas G may or may not be in a sterile state. Besides, the internal pressure (gas pressure) of the bomb 14 is preferably not less than 0.05 MPa, more preferably in the range from 0.09 MPa to 0.11 MPa.

At an intermediate portion of the tube 13, there is disposed a filter 16 housed in a housing 15. The filter 16 can trap foreign matter mixed into the gas G, before the gas G is supplied to the applicator 100.

As shown in FIGS. 1 to 5, the structure 7 is elongate in shape, and extends distally from the base portion 4. As aforementioned, the structure 7 includes the outer tube 5 and the inner tubes 6a and 6b, and further includes a gas-permeable tube 9.

The outer tube 5 includes a tube main body 51 which is tubular in shape, and a nozzle head 52 provided at a distal portion of the tube main body 51.

The tube main body 51 has a proximal portion supported on the base portion 4, and communicates with the tube 13 connected to the base portion 4. This enables the gas G to be supplied to the outer tube 5. The tube main body 51 is so configured that a gap is formed between the inner tubes 6a and 6b which will be described later and the gas-permeable tube 9, and the gas G can flow down through the gap. Thus, the outer tube 5 functions as a gas channel 53 through which the gas G flows down.

As shown in FIGS. 2 to 5, the tube main body 51 is provided at a distal portion thereof with the nozzle head 52. The nozzle head 52 is tubular in shape, and a distal opening thereof constitutes a jet port 521 from which the mixed liquid L3 is jetted. The aperture diameter of the jet port 521 is smaller than the inside diameter of the outer tube 5. This ensures that the mixed liquid L3 is jetted energetically, to securely reach a target region.

While the nozzle head 52 is composed as a separate body from the tube main body 51 in the present embodiment, the nozzle head 52 may be formed integrally with the tube main body 51.

Such an outer tube 5 is provided at a distal portion thereof with a reduced diameter portion 54 which is reduced in inside diameter. To the reduced diameter portion 54, a distal portion of the gas-permeable tube 9 is secured. Besides, that space in the reduced diameter portion 54 which is located downstream of the gas-permeable tube 9 constitutes a confluence portion 55 in which the flows of the first liquid L1 and the second liquid L2 join. In the confluence portion 55, the first liquid L1 and the second liquid L2 are mixed with each other, whereby the mixed liquid L3 is prepared.

In addition, as shown in FIG. 3, the reduced diameter portion 54 is provided, at its portion corresponding to the confluence portion 55, with a stepped portion 58 at which its inside diameter is reduced sharply. The first liquid L1 and the second liquid L2 flowing into the confluence portion 55 together with the gas G are divided into a portion which collides against the stepped portion 58 and reflected in the direction of arrow C in FIG. 3 and a portion which does not collide against the stepped portion 58 but flows directly toward the jet port 521 (in the direction of arrow D in FIG. 3), and these two portions collide against each other inside the confluence portion 55. As a result, the first liquid L1 and the second liquid L2 are agitated and are mixed sufficiently with each other. Accordingly, the mixed liquid L3 in a uniform state can be obtained.

Besides, in the applicator 100, the outer tube 5 can be divided into a rigid portion 56 and a flexible portion 57 on the distal side of the rigid portion 56.

The rigid portion 56 is a portion which accounts for not less than 60% of the outer tube 5 and which is formed from one of various metallic materials such as, for example, stainless steel, aluminum, copper, copper alloys, etc. This ensures that the posture of the nozzle 3 as a whole can be maintained. In other words, the nozzle 3 as a whole can be prevented from bending. Note that the sheath 8 is preferably formed from a constituent material similar to that of the rigid portion 56, a resin material such as low-friction high-density polyethylene, or a composite material thereof.

The flexible portion 57 is a portion formed, for example, from one of various thermoplastic elastomers based on polyvinyl chloride, polyurethane, polyamide, polyester or the like. The flexible portion 57 tends to be curved in a natural state where no external force is applied thereto. When the tubular sheath 8 covering the outer tube 5 is advanced in the direction of arrow A in FIG. 2, the flexible portion 57 can be corrected into a rectilinear shape, resulting in that the jet port 521 faces forward (distally). When the sheath 8 is retracted in the direction of arrow B in FIG. 2, the flexible portion 57 is released from the correction, to be curved, resulting in that the jet port 521 faces obliquely forward (distally) or sideways, as indicated by alternate long and two short dashes line in FIG. 2. Thus, in the applicator 100, by an operation to move the sheath 8, the orientation of the jet port 521 can be changed according to the position of a target region.

In such an outer tube 5 as this, the inner tube 6a and the inner tube 6b are accommodated. As shown in FIGS. 3 to 5, the inner tube 6a is greater than the inner tube 6b in diametric size, specifically, greater than the inner tube 6b in inside diameter and outside diameter.

The inner tube 6a has a proximal portion connected to the mouth portion 22 of the syringe 1a through the connection portion 41a of the base portion 4. This permits the first liquid L1 to flow down through the inside of the inner tube 6a. Thus, the inside of the inner tube 6a functions as a first liquid channel 61 through which the first liquid L1 flows down. Similarly, the inner tube 6b has a proximal portion connected to the mouth portion 22 of the syringe 1b through the connection portion 41b of the base portion 4. This allows the second liquid L2 to flow down through the inside of the inner tube 6b. Thus, the inside of the inner tube 6b functions as a second liquid channel 62 through which the second liquid L2 flows down.

In addition, distal portions of the inner tubes 6a and 6b are close to each other, and are independently connected to the gas-permeable tube 9.

As depicted in FIG. 6, the gas-permeable tube 9 is tubular in shape, and its cross-sectional shape is circular over its entire length. The gas-permeable tube 9 is a double lumen tube which is formed therein with two lumens. The lumens are circular in cross-sectional shape, and individually function as a first independent channel 91 and a second independent channel 92. The first independent channel 91 is greater than the second independent channel 92 in diametric size.

In addition, in the cross-sectional shape of the gas-permeable tube 9, the sum of the areas relevant to the first independent channel 91 and the second independent channel 92 preferably accounts for 5% to 50%, more preferably 20% to 30%, of the total area of the cross-sectional shape of the gas-permeable tube 9. With the gas-permeable tube 9 sized (in diameter) to satisfy the above-mentioned numerical range, the gas-permeable tube 9 can be made comparatively small in diametric size (thickness) while sufficiently securing the first independent channel 91 and the second independent channel 92. As a result, the nozzle 3 can be made comparatively small in diametric size (thickness). Accordingly, the nozzle 3 can be easily inserted into a trocar tube which is used in a laparoscopic operation and which is comparatively small in diameter, so that an applying operation can be conducted directly in that state.

Besides, in the first independent channel 91, a distal portion of the inner tube 6a is fitted liquid-tight. This ensures that the first independent channel 91 and the first liquid channel 61 communicate with each other, and the first liquid L1 can flow down through the first independent channel 91. On the other hand, a distal portion of the inner tube 6b is inserted liquid-tight in the second independent channel 92. This ensures that the second independent channel 92 and the second liquid channel 62 communicate with each other, and the second liquid L2 can flow down through the second independent channel 92. Note that as aforementioned, the first liquid L1 is higher than the second liquid L2 in viscosity, but the first liquid L1 can flow down through the first independent channel 91 easily, since the first independent channel 91 is greater in diameter than the second independent channel 92.

Thus, the gas-permeable tube 9 is supported at both ends thereof by the reduced diameter portion 54 and by the inner tubes 6a and 6b. By this, the gas-permeable tube 9 is prevented from positionally deviating in the radial direction of the nozzle 3 during use of the applicator 100.

The gas-permeable tube 9 is composed of a gas-permeable membrane 93. The gas-permeable membrane 93 is formed with a multiplicity of pores (not depicted). Each of the pores pierces the gas-permeable membrane 93 in the thickness direction. The average pore diameter of the pores is not particularly limited, and is preferably not more than 2 pm, for example. In addition, the gas-permeable membrane 93 has impermeability to the first liquid L1 and the second liquid L2 (water repellency), specifically, hydrophobicity.

Owing to such a configuration as above, the gas-permeable membrane 93 is permeable to the gas G but is impermeable to the first liquid L1 and the second liquid L2. This allows the gas G to flow into the first independent channel 91 and the second independent channel 92 through the gas-permeable membrane 93. In this case, the gas G having flowed into the first independent channel 91 flows downstream together with the first liquid L1 flowing downstream, and the gas G having flowed into the second independent channel 92 flows downstream together with the second liquid L2 flowing downstream. Therefore, the first liquid L1 and the second liquid L2 flow into the confluence portion 55 together with the gas G, and are mixed with each other to be the mixed liquid L3, which is jetted from the jet port 521 together with the gas G.

In addition, as shown in FIG. 2, a length S1 of the first independent channel 91 and the second independent channel 92 is greater than a length S2 of the confluence portion 55 in the longitudinal direction of the nozzle 3. This ensures that the gas G can sufficiently flow into the first independent channel 91 and the second independent channel 92. Therefore, the mixed liquid L3 which is jetted is securely turned into a mist, and the mixed liquid L3 can be applied to a wide area. Further, the confluence portion 55 can be sufficiently spaced apart from the first liquid channel 61 and the second liquid channel 62. Consequently, the mixed liquid L3 can be securely prevented from flowing back into the first liquid channel 61 and the second liquid channel 62 from the confluence portion 55.

Besides, in the present invention, the confluence portion 55 is smaller (shorter) than in the case where the inside of the gas-permeable tube 9 constitutes a confluence portion, but the mixed liquid L3 in a uniform state can be obtained owing to the stepped portion 58, as aforementioned.

In addition, as shown in FIG. 6, a thickness T of a tube wall located between the first independent channel 91 and the second independent channel 92 is preferably 3% to 30%, more preferably 8% to 13%, of a diameter D (outside diameter) of the gas-permeable tube 9. This permits the thickness T to be as small as possible, and, as the thickness T is smaller, the first independent channel 91 and the second independent channel 92 can be made greater in size accordingly. Therefore, the gas G and the first liquid are mixed more uniformly in the first independent channel 91, and the gas G and the second liquid L2 are mixed more uniformly in the second independent channel 92. Since the first liquid L1 and the second liquid L2 flow into the confluence portion 55 in the state of being individually mixed with the gas G uniformly, the mixed liquid L3 in a more uniform state can be obtained.

Besides, as aforementioned, the gas-permeable tube 9 is circular in cross-sectional shape over its entire length. Therefore, the gas G can flow into the first independent channel 91 and the second independent channel 92 through any part of the gas-permeable membrane 93 in the circumferential direction. As a result, the gas G can be supplied into the first independent channel 91 and the second independent channel 92 in a proper quantity. Through the first independent channel 91 and the second independent channel 92, therefore, the gas G can be supplied also into the confluence portion 55 in a proper quantity. Consequently, the mixed liquid L3 which is jetted is reliably turned into a mist.

Note that when the jetting of the mixed liquid L3 is stopped, as shown in FIG. 4, the gas G having flowed in through the gas-permeable membrane 93 flows through the first independent channel 91 and the second independent channel 92 into the confluence portion 55, so that the mixed liquid L3 in the confluence portion 55 is assuredly pushed outward (blown away) by the gas G. As a result, the mixed liquid L3 is prevented from remaining in the confluence portion 55. Accordingly, the mixed liquid L3 can be prevented from solidifying to cause clogging of the jet port 521. In addition, a residual liquid of the mixed liquid L3 can be reliably prevented from leaking from the jet port 521.

Note that the configuration for providing the gas-permeable membrane 93 with hydrophobicity is not specifically restricted. For example, a hydrophobic material (e.g., polytetrafluoroethylene) may be used as the constituent material of the gas-permeable membrane 93, or a surface of the gas-permeable membrane 93 may be subjected to a treatment for making it hydrophobic (e.g., a plasma treatment).

A distal portion of the gas-permeable tube 9 as above is inserted in the reduced diameter portion 54 of the outer tube 5, and is secured gas-tight to the latter through an adhesive (fixing material) 17 (see FIGS. 3 to 6). According to such a configuration as this, the confluence portion 55 and the gas channel 53 can be separated from each other by the reduced diameter portion 54. In other words, the reduced diameter portion 54 functions as a partition wall portion for separating the gas channel 53 and the confluence portion 55 from each other. As a result, the gas G can be prevented from flowing alone directly into the confluence portion 55. Furthermore, the partition wall portion functions also as a support portion for supporting the gas-permeable tube 9.

In addition, as shown in FIGS. 2 to 5, since the gas-permeable tube 9 is inserted in the reduced diameter portion 54, the distal ends of the first independent channel 91 and the second independent channel 92 are located accordingly downstream of the distal of the gas channel 53. As a result, the confluence portion 55 and the distal of the gas channel 53 can be spaced apart from each other along the longitudinal direction of the nozzle 3. Therefore, flowing of the gas G alone into the confluence portion 55 can be prevented more assuredly.

As shown in FIG. 3, a length S3 of that portion of the gas-permeable tube 9 which is secured (fixed) to the reduced diameter portion 54 is preferably 5% to 500%, more preferably 40% to 150%, of the length S4 of the reduced diameter portion 54. This ensures that a sufficient adhesion area between the gas-permeable tube 9 and the reduced diameter portion 54 can be secured, and the size of the confluence portion 55 in which the first liquid L1 and the second liquid L2 are mixed can be made sufficiently large. Therefore, a sufficient bond strength can be obtained between the gas-permeable tube 9 and the reduced diameter portion 54. Further, the first liquid L1 and the second liquid L2 can be sufficiently mixed with each other in the confluence portion 55, so that the mixed liquid L3 in a uniform state can be obtained.

Note that the adhesive 17 is not specifically restricted, and, for example, an acrylic ultraviolet-curing type adhesive can be used.

Besides, that part of the gas-permeable tube 9 which makes contact with the adhesive 17 is preferably subjected beforehand to a surface treatment for enhancing adhesion between the part and the adhesive 17. The surface treatment is not specifically restricted. Examples of the surface treatment applicable include a plasma treatment, a primer treatment (for example, application of silane or titanate coupling agent), and application of a metallic sodium-containing fluororesin surface treatment agent (e.g., "TETRA-ETCH A" or "TETRA-ETCH B" (TETRA-ETCH is a registered trademark) produced by Junkosha Inc.).

Now, an operating state of the applicator 100 will be described.
[1] First, as shown in FIG. 2, the applicator 100 with the bomb 14 connected thereto is prepared. Then, prior to an applying operation, the valve 141 of the bomb 14 is put into an open state, to preliminarily supply the gas G to the applicator 100. This causes the gas G to flow, in the nozzle 3, sequentially through the gas channel 53, the first independent channel 91 and the second independent channel 92, and the confluence portion 55, to jet from the jet port 521.
[2] Next, an index finger and a middle finger of one hand are put on the flange portion 23 of the syringe outer cylinder 2, and a thumb is put on the flange portion 113 of the plunger 11. Thereafter, the jet port 521 of the nozzle 3 is directed toward a target region. In this state, then, a force is applied with the thumb, to push the plunger 11 toward the distal side, thereby performing an applying operation. As a result, as shown in FIG. 3, the first liquid L1 is supplied into the first liquid channel 61, and the second liquid L2 is supplied into the second liquid channel 62.

When the plunger 11 is further pushed continuedly, the first liquid L having flowed down through the first liquid channel 61 is caused by the pushing force to flow down through the first independent channel 91 in the gas-permeable tube 9, while the second liquid L2 having flowed down through the second liquid channel 62 is caused by the pushing force to flow down through the second independent channel 92 in the gas-permeable tube 9.

In this instance, as depicted in FIG. 3, the gas G is flowing into the first independent channel 91 and the second independent channel 92 by permeating the gas-permeable membrane 93. This results in that the gas G is mixed with the first liquid L1 in the first independent channel 91, and is mixed with the second liquid L2 in the second independent channel 92, before flowing into the confluence portion 55.

As illustrated in FIG. 3, in the confluence portion 55, the first liquid L1 and the second liquid L2 having flowed down together with the gas G are mixed with each other, to be the mixed liquid L3. The mixed liquid L3 is jetted from the jet port 521 together with the gas G, while being turned into a mist, and is applied to the target region. Thus, in this applicator 100, the mixed liquid L3 is jetted by the pushing force exerted on the plunger 11 and the gas pressure of the gas G.

[3] After a predetermined amount of the mixed liquid L3 is applied to the target region, the pushing force exerted on the plunger 11 is relaxed, to stop the applying operation. As a result, the supply of the first liquid L1 into the first independent channel 91 is stopped, and the supply of the second liquid L2 into the second independent channel 92 is stopped. In this supply stopped state in which the supply of the liquids is stopped, the first liquid L1 is remaining in the first independent channel 91, and the second liquid L2 is remaining in the second independent channel 92.

Besides, in the supply stopped state, the gas G is flowing into the first independent channel 91 and the second independent channel 92 in a continued manner. As a consequence, the first liquid L1 in the first independent channel 91 and the second liquid L2 in the second independent channel 92 flow into the confluence portion 55, to be the mixed liquid L3, which is jetted from the jet port 521 (see FIG. 4).

Note that in the confluence portion 55, the mixed liquid L3 might tend to remain as a residual liquid. In the present invention, however, the confluence portion 55 is located downstream of the first independent channel 91 and the second independent channel 92, so that the mixed liquid L3 in the confluence portion 55 can directly be blown downstream in a reliable manner by the gas G flowing into the confluence portion 55 from the upstream side (see FIG. 5). Therefore, the mixed liquid L3 in the confluence portion 55 can be securely jetted from the jet port 521. Accordingly, clogging of the jet port 521 with the mixed liquid L3 can be prevented assuredly. As a result, the applying operation can be performed again.

Furthermore, the gas G flows into the confluence portion 55 only from the first independent channel 91 and the second independent channel 92. With this configuration, the mixed liquid L3 in the confluence portion 55 can be securely prevented from flowing back against the gas pressure of the gas G which flows down from the first independent channel 91 and the second independent channel 92. Accordingly, the mixed liquid L3 in the confluence portion 55 can be prevented from flowing back into the first independent channel 91 and the second independent channel 92. Consequently, clogging of the first independent channel 91 and the second independent channel 92 can also be prevented assuredly.

While the applicator of the present invention has been described with reference to the embodiment illustrated in the drawings, the invention is not limited to this. Each of the components of the applicator can be replaced with one having an arbitrary configuration that can exhibit the same or similar function to the original. Besides, arbitrary structures may be added.

Note that the number of the inner tubes has been two in the present embodiment, this is not limitative. For example, a single inner tube or three or more inner tubes may be provided. Besides, where only one inner tube is provided, the inner tube is configured from a double lumen tube or a tube having three or more lumens.

### Industrial Applicability

The applicator of the present invention includes a nozzle, the nozzle including a gas channel through which a gas flows down, a plurality of liquid channels which are provided inside the gas channel and through which liquids flow down, and a confluence portion which is located on a downstream side of the liquid channels and at which the liquid channels join,
wherein of the gas channel, a portion between the liquid channels and the confluence portion is configured from a single gas-permeable tube composed of a gas-permeable membrane permeable to the gas but impermeable to the liquids, and
the gas-permeable tube includes a plurality of independent channels which are independently connected to the liquid channels and through which the liquids flowing down from the liquid channels flow toward the confluence portion together with the gas flowing in through the gas-permeable membrane. Therefore, when an applying operation is stopped, the mixed liquid obtained by mixing of the liquids can be prevented from flowing back.

Accordingly, the applicator of the present invention has industrial applicability.

### Description of Reference Symbols

- 100: Applicator
- 10: Syringe assembly
- 1a, 1b: Syringe
- 2: Syringe outer cylinder
- 21: Barrel portion
- 211: Distal wall portion
- 22: Mouth portion (Syringe side port portion)
- 23: Flange portion
- 3: Nozzle
- 4: Base portion
- 41a, 41b, 42: Connection portion
- 5: Outer tube
- 51: Tube main body
- 52: Nozzle head
- 521: Jet port
- 53: Gas channel
- 54: Reduced diameter portion
- 55: Confluence portion
- 56: Rigid portion
- 57: Flexible portion
- 58: Stepped portion
- 6a, 6b: Inner tube
- 61: First liquid channel
- 62: Second liquid channel
- 7: Structure
- 8: Sheath
- 9: Gas-permeable tube
- 91: First independent channel
- 92: Second independent channel
- 93: Gas-permeable membrane
- 11: Plunger
- 111, 112: Plunger portion
- 113: Flange portion
- 12: Gasket
- 13: Tube
- 14: Bomb
- 141: Valve (Cock)
- 15: Housing
- 16: Filter
- 17: Adhesive
- G: Gas
- L1: First liquid
- L2: Second liquid
- L3: Mixed liquid
- S1, S2, S3, S4: Length
- D: Diameter
- T: Thickness

## Claims

1. An applicator (100), pre-filled with two separated liquids (L1, L2), the first liquid (L1) and the second liquid (L2) being configured to become a gel when being mixed with each other, the applicator comprising a nozzle (3), the nozzle (3) including a gas channel (53) through which a gas may flow down, two liquid channels (61, 62) which are provided inside the gas channel (53) and through which said liquids (L1, L2) may respectively flow down, and a confluence portion (55) which is located on a downstream side of the liquid channels (61, 62) and in which the flows of the first liquid (L1) and the second liquid (L2) join, wherein a portion between the liquid channels (61, 62) and the confluence portion (55) is configured from a single gas-permeable tube (9) composed of a gas-permeable membrane (93) permeable to the gas but impermeable to the liquids, and **characterised in that** the gas-permeable tube (9) includes a plurality of independent channels (91, 92) which are independently connected to the liquid channels (61, 62) and through which the liquids flowing down from the liquid channels (61, 62) flow toward the confluence portion (55) together with gas when flowing in through the gas-permeable membrane (93), and **in that** a length (S1) of the independent channels (91, 92) is greater than a length (S2) of the confluence portion (55) in a longitudinal direction of the nozzle (3).

2. The applicator (100) according to claim 1, wherein the cross-sectional area of the independent channels (91, 92) of the gas-permeable tube (9) is 5% to 50% of of the cross-sectional area of the gas-permeable tube (9) .

3. The applicator (100) according to claim 1 or 2, wherein a thickness (T) of a wall portion located between the adjacent independent channels (91, 92) is 3% to 30% of an outside diameter (D) of the gas-permeable tube (9).

4. The applicator (100) according to any one of claims 1 to 3, wherein the liquids flowing down through the plurality of liquid channels differ from each other in viscosity,
the plurality of independent channels (91, 92) differ from each other in diametric size, and
the liquid (L1) having a higher viscosity flows down through the independent channel having a greater diametric size whereas the liquid (L2) having a lower viscosity flows down through the independent channel having a smaller diametric size.

5. The applicator (100) according to any one of claims 1 to 4, wherein the nozzle (3) has a double tube structure having a plurality of inner tubes and an outer tube accommodating the inner tubes, the inner tubes function as the liquid channels (61, 62), and a gap between the inner tubes and the outer tube functions as the gas channel (53).

6. The applicator (100) according to claim 5,
wherein the outer tube has a partition wall portion separating the gas channel (53) and the confluence portion (55) from each other, and
the partition wall portion constitutes a support portion supporting the gas-permeable tube (9) .

7. The applicator (100) according to claim 5 or 6,
wherein the outer tube has a reduced diameter portion reduced in inside diameter at an end portion on a downstream side thereof, and
a space inside the reduced diameter portion constitutes the confluence portion (55).

8. The applicator (100) according to claim 7, wherein the gas-permeable tube (9) has an outer peripheral portion on a downstream side thereof fixed to the reduced diameter portion through a fixing material fixing them in a gas-tight manner.

9. The applicator (100) according to any one of claims 1 to 8, comprising
liquid supplying means configured to supply the liquids to the nozzle (3),
wherein the liquid supplying means is a syringe (1a, 1b) including a syringe outer cylinder (2), a gasket (12) inserted in the syringe outer cylinder (2), a pusher operated to move the gasket(12) in a longitudinal direction of the syringe outer cylinder (2), and liquid filled in a space defined by the syringe outer cylinder and the gasket (12).

## Patentansprüche

1. Applikator (100), vorgefüllt mit zwei getrennten Flüssigkeiten (L1, L2), wobei die erste Flüssigkeit (L1) und die zweite Flüssigkeit (L2) dazu ausgebildet sind, ein Gel zu werden, wenn sie miteinander gemischt werden, wobei der Applikator eine Düse (3) umfasst, wobei die Düse (3) einen Gaskanal (53) aufweist, durch den ein Gas nach unten strömen kann, zwei Flüssigkeitskanäle (61, 62), die in dem Gaskanal (53) vorgesehen sind und durch die die Flüssigkeiten (L1, L2) jeweils nach unten strömen können, und einen Konfluenzabschnitt (55), der sich auf einer stromabwärtigen Seite der Flüssigkeitskanäle (61, 62) befindet und in dem die Ströme der ersten Flüssigkeit (L1) und der zweiten Flüssigkeit (L2) zusammentreffen, wobei ein Abschnitt zwischen den Flüssigkeitskanälen (61, 62) und dem Konfluenzabschnitt (55) aus einem einzigen gasdurchlässigen Rohr (9) gebildet ist, das aus einer gasdurchlässigen Membran (93) besteht, die für das Gas durchlässig, aber für die Flüssigkeiten undurchlässig ist, und **dadurch gekennzeichnet, dass** das gasdurchlässige Rohr (9) eine Vielzahl von unabhängigen Kanälen (91, 92) aufweist, die unabhängig voneinander mit den Flüssigkeitskanälen (61, 62) verbunden sind und durch die die von den Flüssigkeitskanälen (61, 62) nach unten strömenden Flüssigkeiten zusammen mit dem Gas zu dem Konfluenzabschnitt (55) strömen, wenn sie durch die gasdurchlässige Membran (93) einströmen, und dass eine Länge (S1) der unabhängigen Kanäle (91, 92) größer ist als eine Länge (S2) des Konfluenzabschnitts (55) in Längsrichtung der Düse (3).

2. Applikator (100) nach Anspruch 1, wobei die Querschnittsfläche der unabhängigen Kanäle (91, 92) des gasdurchlässigen Rohres (9) 5% bis 50% der Querschnittsfläche des gasdurchlässigen Rohres (9) beträgt.

3. Applikator (100) nach Anspruch 1 oder 2, wobei eine Dicke (T) eines zwischen den benachbarten unabhängigen Kanälen (91, 92) befindlichen Wandabschnitts 3% bis 30% eines Außendurchmessers (D) des gasdurchlässigen Rohres (9) beträgt.

4. Applikator (100) nach einem der Ansprüche 1 bis 3,
wobei sich die durch die Vielzahl von Flüssigkeitskanälen nach unten strömenden Flüssigkeiten in ihrer Viskosität voneinander unterscheiden,
die Vielzahl von unabhängigen Kanälen (91, 92) sich in ihrer Durchmessergröße voneinander unterscheiden, und
die Flüssigkeit (L1) mit einer höheren Viskosität durch den unabhängigen Kanal mit einer größeren Durchmessergröße nach unten strömt, während die Flüssigkeit (L2) mit einer niedrigeren Viskosität durch den unabhängigen Kanal mit einer kleineren Durchmessergröße nach unten strömt.

5. Applikator (100) nach einem der Ansprüche 1 bis 4, wobei die Düse (3) als Doppelrohr konstruiert ist, das eine Vielzahl von Innenrohren und ein Außenrohr besitzt, das die Innenrohre aufnimmt, die Innenrohre als Flüssigkeitskanäle (61, 62) fungieren und ein Spalt zwischen den Innenrohren und dem Außenrohr als Gaskanal (53) fungiert.

6. Applikator (100) nach Anspruch 5,
wobei das Außenrohr einen Trennwandabschnitt besitzt, der den Gaskanal (53) und den Konfluenzabschnitt (55) voneinander trennt, und
der Trennwandabschnitt einen Stützabschnitt bildet, der das gasdurchlässige Rohr (9) trägt.

7. Applikator (100) nach Anspruch 5 oder 6,
wobei das Außenrohr einen Abschnitt mit vermindertem Durchmesser besitzt, dessen Innendurchmesser an einem Endabschnitt auf seiner stromabwärtigen Seite verringert ist, und
ein Raum in dem Abschnitt mit vermindertem Durchmesser den Konfluenzabschnitt (55) bildet.

8. Applikator (100) nach Anspruch 7, wobei das gasdurchlässige Rohr (9) einen äußeren Umfangsabschnitt auf seiner stromabwärtigen Seite besitzt, der durch ein Befestigungsmaterial, das sie gasdicht befestigt, an dem Abschnitt mit vermindertem Durchmesser befestigt ist.

9. Applikator (100) nach einem der Ansprüche 1 bis 8, umfassend
ein Flüssigkeitszufuhrmittel, das dazu ausgebildet ist, die Flüssigkeiten der Düse (3) zuzuführen,
wobei das Flüssigkeitszufuhrmittel eine Spritze (1a, 1b) ist, die einen Spritzenaußenzylinder (2), eine in den Spritzenaußenzylinder (2) eingesetzte Dichtung (12), einen Schieber, mit dem die Dichtung (12) in Längsrichtung des Spritzenaußenzylinders (2) bewegt wird, und Flüssigkeit in einem durch den Spritzenaußenzylinder und die Dichtung (12) begrenzten Raum aufweist.

## Revendications

1. Applicateur (100), pré-rempli avec deux liquides séparés (L1, L2), le premier liquide (L1) et le second liquide (L2) étant configurés pour devenir un gel lorsqu'ils sont mélangés l'un avec l'autre, l'applicateur comprenant une buse (3), la buse (3) comportant un canal de gaz (53), à travers lequel peut s'écouler un gaz, deux canaux de liquide (61, 62), qui sont prévus à l'intérieur du canal de gaz (53) et à travers lesquels peuvent s'écouler respectivement lesdits liquides (L1, L2) et une partie formant confluence (55), qui est située sur un côté aval des canaux de liquide (61, 62) et dans lesquels se rejoignent les écoulements du premier liquide (L1) et du second liquide (L2),
dans lequel une partie entre les canaux de liquide (61, 62) et la partie formant confluence (55) est configurée à partir d'un unique tube perméable au gaz (9), composé d'une membrane perméable au gaz (93), perméable au gaz mais imperméable aux liquides et
**caractérisé en ce que**
le tube perméable au gaz (9) comporte une pluralité de canaux indépendants (91, 92), qui sont reliés indépendamment aux canaux de liquide (61, 62) et travers lesquels les liquides, qui s'écoulent depuis les canaux de liquide (61, 62), s'écoulent vers la partie formant confluence (55) avec le gaz, lorsqu'ils affluent à travers la membrane perméable au gaz (93) et **en ce**
**qu'**une longueur (S1) des canaux indépendants (91, 92) est supérieure à une longueur (S2) de la partie formant confluence (55), dans une direction longitudinale de la buse (3).

2. Applicateur (100) selon la revendication 1,
dans lequel la section des canaux indépendants (91, 92) du tube perméable au gaz (9) est de 5 % à 50 % de la section du tube perméable au gaz (9).

3. Applicateur (100) selon la revendication 1 ou 2, dans lequel une épaisseur (T) d'une partie formant paroi, située entre les canaux indépendants (91, 92) adjacents, est de 3 % à 30 % d'un diamètre extérieur (D) du tube perméable au gaz (9).

4. Applicateur (100) selon l'une quelconque des revendications 1 à 3,
dans lequel les liquides, qui s'écoulent à travers la pluralité de canaux de liquides, diffèrent l'un de l'autre en viscosité, la pluralité de canaux indépendants (91, 92) diffèrent l'un de l'autre en dimension diamétrale et le liquide (L1), ayant une viscosité supérieure, s'écoule à travers le canal indépendant ayant une dimension diamétrale supérieure, tandis que le liquide (L2), ayant une viscosité inférieure, s'écoule à travers le canal indépendant ayant une dimension diamétrale plus faible.

5. Applicateur (100) selon l'une quelconque des revendications 1 à 4,
dans lequel la buse (3) a une double structure de tube, ayant une pluralité de tubes intérieurs et un tube extérieur logeant les tubes intérieurs, les tubes intérieurs fonctionnent comme les canaux de liquide (61, 62) et un espace entre les tubes intérieurs et le tube extérieur fonctionne comme le canal de gaz (53).

6. Applicateur (100) selon la revendication 5,
dans lequel le tube extérieur a une partie formant cloison de séparation, qui sépare le canal de gaz (53) et la partie formant confluence (55) l'un de l'autre et
la partie formant cloison de séparation constitue une partie formant support, qui supporte le tube perméable au gaz (9).

7. Applicateur (100) selon la revendication 5 ou 6,
dans lequel le tube extérieur a une partie au diamètre réduit, réduite en diamètre intérieur et une partie d'extrémité sur un côté aval de celui-ci et
un espace à l'intérieur de la partie au diamètre réduit constitue la partie formant confluence (55).

8. Applicateur (100) selon la revendication 7,
dans lequel le tube perméable au gaz (9) a une partie périphérique extérieure, sur un côté aval de celui-ci, fixée à la partie au diamètre réduit par le biais d'un matériau de fixation, qui les fixe d'une manière étanche au gaz.

9. Applicateur (100) selon l'une quelconque des revendications 1 à 8, comprenant un moyen d'alimentation en liquide, configuré pour fournir les liquides à la buse (3),
dans lequel le moyen d'alimentation en liquide est une seringue (1a, 1b), comportant un cylindre extérieur (2) de seringue, un joint (12), inséré dans le cylindre extérieur (2) de seringue, un pousseur, actionné pour déplacer le joint (12) dans une direction longitudinale du cylindre extérieur (2) de seringue et du liquide, chargé dans un espace, défini par le cylindre extérieur de seringue et le joint (12).
